# FASCICULE DE BREVET EUROPEEN

(11) **EP 1 362 864 B1**
(45) Date de publication et mention de la délivrance du brevet: **25.04.2007**
(21) Numéro de dépôt: 03291600.9
(22) Date de dépôt: 30.06.2003
(51) Int. Cl.: C07K 5/02, C07K 5/06, C07D 209/42

(54) **Nouveau procédé de synthèse du perindopril et de ses sels pharmaceutiquement acceptables**
Verfahren für die Synthese von Perindopril und seiner pharmazeutisch geeigneten Salze
Method for synthesis of perindopril and its pharmaceutically acceptable salts

(43) Date de publication de la demande: 19.11.2003
(73) Titulaire: Les Laboratoires Servier, 92415 Courbevoie Cedex (FR)
(72) Inventeur: Dubuffet, Thierry, 76210 Bolbec (FR); Lecouve, Jean-Pierre, 76600 Le Havre (FR)

(56) Documents cités:
- EP-A- 1 321 471
- SUH J T ET AL: "ANGIOTENSIN-CONVERTING ENZYME INHIBITORS: N-SUBSTITUTED GLYCINE DERIVATIVES" EUROPEAN JOURNAL OF MEDICINAL CHEMISTRY, EDITIONS SCIENTIFIQUE ELSEVIER, PARIS, FR, vol. 20, no. 6, 1985, pages 563-570, XP001106728 ISSN: 0223-5234

## Description

La présente invention concerne un procédé de synthèse industrielle du perindopril de formule (I) : 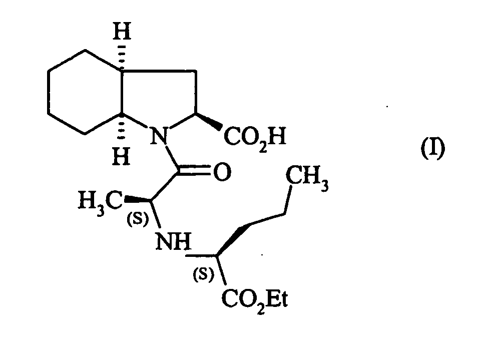 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse industrielle performant, facilement transposable à l'échelle industrielle, conduisant au perindopril avec un bon rendement et une excellente pureté, à partir de matières premières bon marché.
Le brevet EP 0 308 341 décrit la synthèse industrielle du perindopril par couplage de type peptidique de l'ester benzylique de l'acide (2*S*, 3a*S*, 7a*S*)-octahydroindole 2-carboxylique avec l'ester éthylique de la N-[(S)-1-carboxybutyl]-(S)-alanine, suivie de la déprotection du groupement carboxylique de l'hétérocycle par hydrogénation catalytique.

Ce procédé présente l'avantage de conduire au perindopril avec un bon rendement, à partir de matières premières dont la synthèse industrielle est décrite.
Il présente cependant aussi des inconvénients liés à l'utilisation du DCC (dicyclohexylcarbodiimide) dans l'étape de couplage : formation d'impuretés de couplage, ainsi que de dicyclohexylurée, un sous-produit difficile à éliminer.

Le brevet EP 1 321 471 décrit un procédé de synthèse du perindopril par couplage peptidique de l'ester benzylique de l'acide hexahydroindole carboxylique en présence de DCC, qui présente de ce fait les mêmes inconvénients. La publication Eur. J, Med. Chem 1985, Vol. 20(6), pp. 563-570 décrit l'utilisation d'un dérivé d'oxazolidine dans une étape de couplage comportant par ailleurs une fonction acide protégée.

La Demanderesse a présentement mis au point un nouveau procédé de synthèse industrielle du perindopril à partir d'acide hexahydroindole carboxylique non protégé, qui évite la formation des produits secondaires liés à l'utilisation de DCC.

Plus spécifiquement, la présente invention concerne un procédé de synthèse industrielle du perindopril, et de ses sels pharmaceutiquement acceptables, caractérisé en ce que l'on met en réaction le composé de formule (II) : 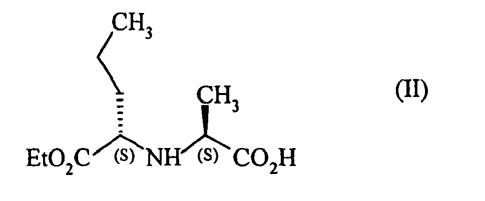 avec un composé de formule (III) : 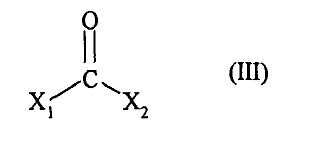 dans laquelle X₁ et X₂, identiques ou différents, représentent chacun un groupement partant,
pour conduire au composé de formule (IV) : 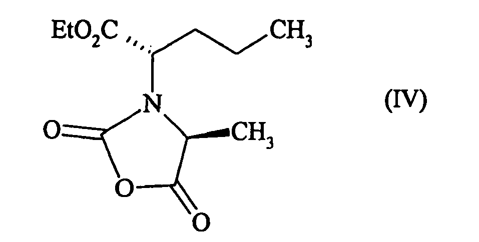 que l'on met en réaction avec un composé de formule (V) : 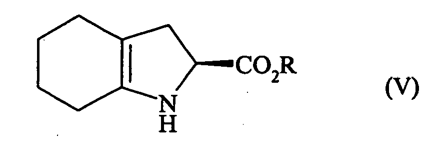 dans laquelle R représente un atome d'hydrogène,
ou son sel d'addition à un acide minéral ou organique,
pour conduire après isolement au composé de formule (VI) : 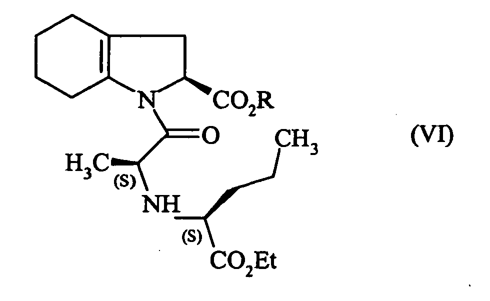 dans laquelle R est tel que défini précédemment,
que l'on hydrogène en présence d'un catalyseur tel que, par exemple, le palladium, le platine, le rhodium ou le nickel,
sous une pression d'hydrogène comprise entre 1 et 30 bars, de préférence entre 1 et 10 bars, pour conduire au perindopril de formule (I), que l'on transforme, si on le souhaite, en un sel pharmaceutiquement acceptable tel que le sel de tert-butylamine.

Parmi les groupements partants X₁ et X₂ appropriés, on peut citer à titre non limitatif les atomes d'halogène et les groupements tosylate, mésylate, alkoxy (C₁-C₆) linéaire ou ramifié, alkylthio (C₁-C₆) linéaire ou ramifié, imidazolyle, benzimidazolyle, tétrazolyle, benzotétrazolyle, trihaloalkyle (C₁-C₆) linéaire ou ramifié, trihaloalkoxy (C₁-C₆) linéaire ou ramifié et succinimidyloxy.
Parmi les groupements partants X₁ et X₂ préférés, on peut citer l'atome de chlore et les groupements imidazolyle et trichlorométhoxy.

L'exemple ci-dessous illustre l'invention, mais ne la limite en aucune façon.

### EXEMPLE : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : (2S)-2-[(4S)-4-Méthyl-2,5-dioxo-1,3-oxazolidin-3-yl]-pentanoate d'éthyle

Dans un réacteur, charger à 0°C 200 g de N-[(S)-carbéthoxy-1-butyl]-(S)-alanine, 1,5 1 de toluène, puis 184 g de 1,1'-carbonyl-diimidazole, puis amener la température du milieu réactionnel à 20°C. Après 1 heure d'agitation à 20°C, refroidir à nouveau le mélange à 0°C, filtrer le précipité obtenu, puis évaporer le filtrat, pour conduire au produit du titre avec un rendement de 90 %.

### Stade B : Acide (2S)-1-{(2S)-2-[(1S)-1-(éthoxycarbonyl)-butylamino]propionyl}-2,3,4,5,6,7-hexahydro-1H-indole-2-carboxylique

Dans un réacteur, placer 200 g d'acide (2S)-2,3 ,4,5 ,6,7-hexahydro- 1H-indole-2-carboxylique et 1,5 1 de dichlorométhane, puis 180 ml de triéthylamine.
Ajouter ensuite lentement une solution de 290 g du composé obtenu au stade précédent dans 500 ml de dichlorométhane, puis agiter 1 heure supplémentaire à température ambiante.

Après addition d'eau, le mélange réactionnel est ensuite refroidi à 15°C et le pH est amené à 4,2 par addition d'une solution d'acide chlorhydrique 2N. Après extraction, les phases organiques sont lavées puis évaporées pour conduire au produit attendu.

### Stade C : Acide (2S, 3aS, 7aS)-1-{(2S)-2-[(1S)-1-(éthoxycarbonyl)-butylamino]-propionyl }-octahydro-1H-indole- 2 -carboxylique

Dans un hydrogénateur, placer 200 g du composé obtenu dans le stade précédent, en solution dans l'acide acétique, puis 5 g de Pt/C à 10 %. Hydrogéner sous pression de 5 bars à température ambiante, jusqu'à absorption de la quantité théorique d'hydrogène.
Eliminer le catalyseur par filtration, puis refroidir entre 0 et 5°C et récolter le solide obtenu par filtration. Laver le gâteau et le sécher jusqu'à poids constant.

### Stade D : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

Le lyophilisat obtenu dans le stade précédent (200 g) est mis en solution dans 2,8 1 d'acétate d'éthyle, puis 40 g de tert-butylamine et 0,4 1 d'acétate d'éthyle sont ajoutés.
La suspension obtenue est ensuite portée au reflux jusqu'à dissolution totale, puis la solution obtenue est filtrée à chaud et refroidie sous agitation jusqu'à une température de 15-20°C.
Le précipité obtenu est alors filtré, réempâté à l'acétate d'éthyle, séché puis broyé pour conduire au produit attendu avec un rendement de 95 %.

## Revendications

1. Procédé de synthèse industrielle du perindopril de formule (I) : 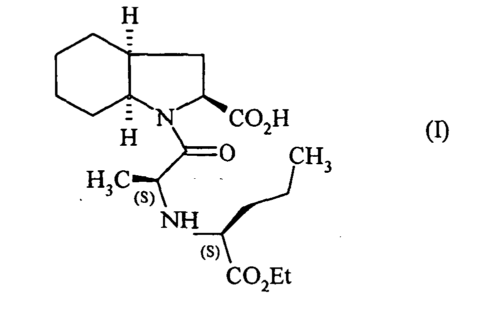 et de ses sels pharmaceutiquement acceptables,
**caractérisé en ce que** l'on met en réaction le composé de formule (II) : 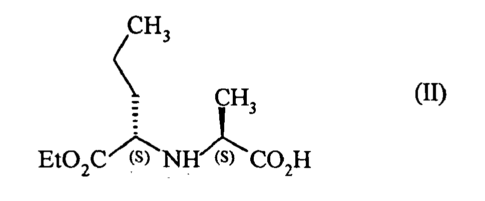 avec un composé de formule (III) : 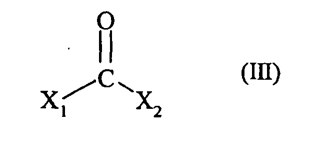 dans laquelle X₁ et X₂, identiques ou différents, représentent chacun un groupement partant,
pour conduire au composé de formule (IV) : 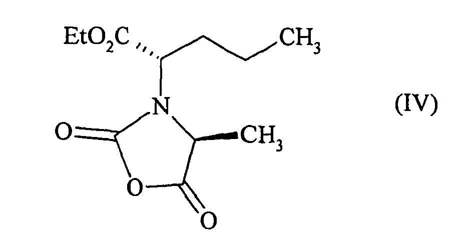 que l'on met en réaction avec un composé de formule (V) : 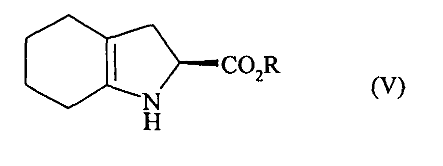 dans laquelle R représente un atome d'hydrogène,
ou son sel d'addition à un acide minéral ou organique,
pour conduire après isolement au composé de formule (VI) : 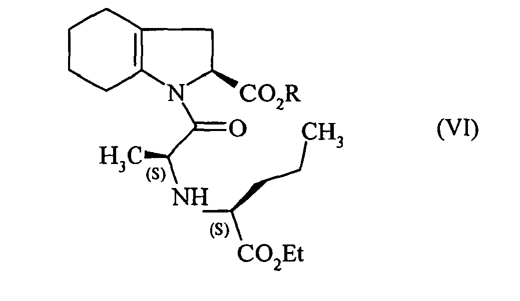 dans laquelle R est tel que défini précédemment,
que l'on hydrogène en présence d'un catalyseur tel que, par exemple, le palladium, le platine, le rhodium ou le nickel,
sous une pression d'hydrogène comprise entre 1 et 30 bars, pour conduire, au perindopril de formule (I), que l'on transforme, si on le souhaite, en un sel pharmaceutiquement acceptable tel que le sel de tert-butylamine.

2. Procédé de synthèse selon la revendication 1, **caractérisé en ce que** la pression d'hydrogène de la réaction d'hydrogénation est comprise entre 1 et 10 bars.

3. Procédé de synthèse selon l'une quelconque des revendications 1 ou 2, **caractérisé en ce que** X₁ et X₂ représentent chacun un atome de chlore ou un groupement imidazolyle ou trichlorométhoxy.

4. Procédé de synthèse selon l'une quelconque des revendications 1 à 3 du perindopril sous sa forme de sel de tert-butylamine.

## Claims

1. Process for the industrial synthesis of perindopril of formula (I) : 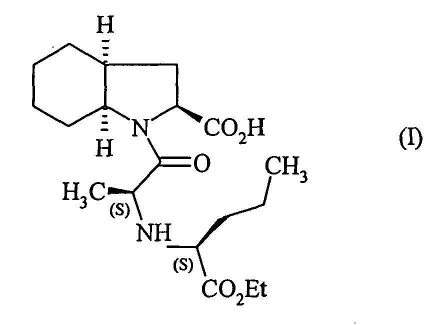 and its pharmaceutically acceptable salts,
**characterised in that** the compound of formula (II) : 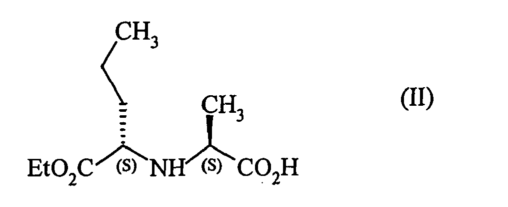 is reacted with a compound of formula (III) : 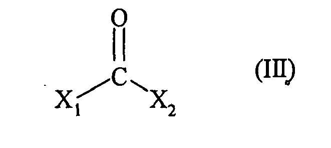 wherein X₁ and X₂, which may be identical or different, each represents a leaving group, to yield the compound of formula (IV) : 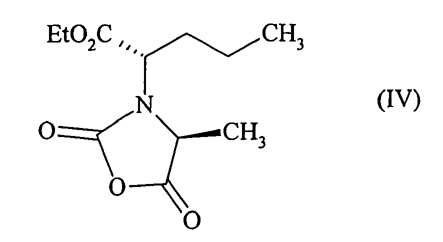 which is reacted with a compound of formula (V) : 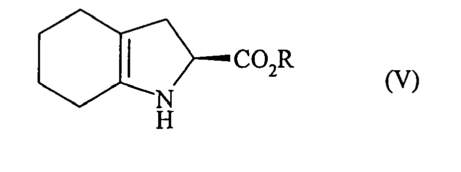 wherein R represents a hydrogen atom,
or an addition salt thereof with a mineral or organic acid,
to yield, after isolation, a compound of formula (VI) : 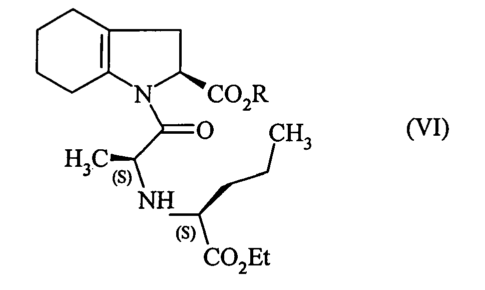 wherein R is as defined hereinbefore,
which is hydrogenated in the presence of a catalyst such as, for example, palladium, platinum, rhodium or nickel,
under a hydrogen pressure of from 1 to 30 bars, to yield perindopril of formula (I) which is converted, if desired, to a pharmaceutically acceptable salt such as the tert-butylamine salt.

2. Synthesis process according to claim 1, **characterised in that** the hydrogen pressure in the hydrogenation reaction is from 1 to 10 bars.

3. Synthesis process according to claim 1 or claim 2, **characterised in that** X₁ and X₂ each represent a chlorine atom or an imidazolyl or trichloromethoxy group.

4. Process according to any one of claims 1 to 3 for the synthesis of perindopril in the form of its tert-butylamine salt.

## Patentansprüche

1. Verfahren zur technischen Synthese von Perindopril der Formel (I): 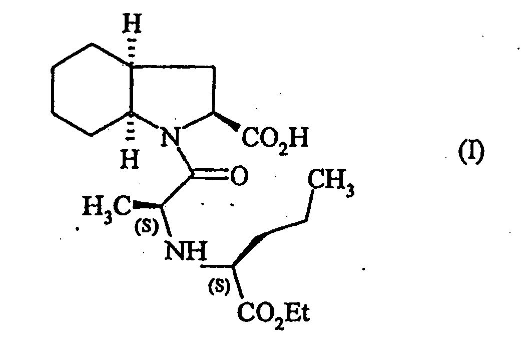 und von seinen pharmazeutisch annehmbaren Salzen,
**dadurch gekennzeichnet, dass** man die Verbindung der Formel (II): 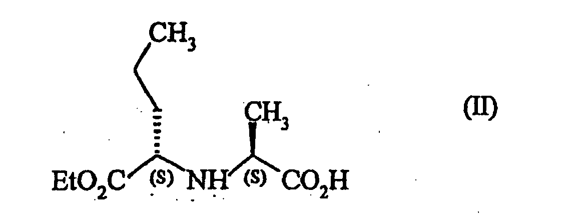 mit einer Verbindung der Formel (III): 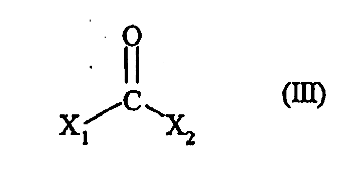 in der X₁ und X₂, die gleichartig oder verschieden sind, jeweils eine austretende Gruppe bedeuten,
umsetzt zur Bildung der Verbindung der Formel (IV): 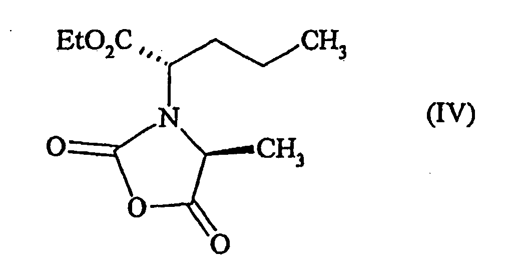 welche man mit einer Verbindung der Formel (V): 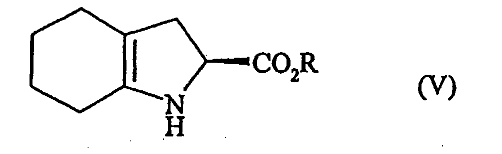 in der R ein Wasserstoffatom bedeutet,
oder seinen Säureadditionssalzen mit einer anorganischen oder organischen Säure umsetzt, sodass man nach der Isolierung die Verbindung der Formel (VI) erhält: 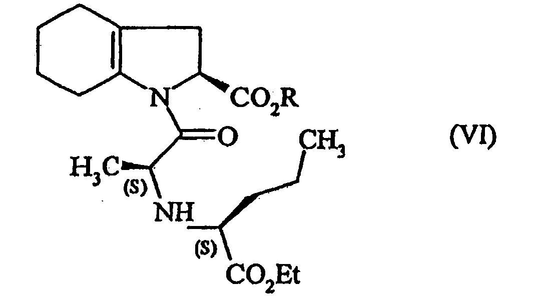 in der R die oben angegebenen Bedeutungen besitzt,
welche man in Gegenwart eines Katalysators, wie beispielsweise Palladium, Platin, Rhodium oder Nickel,
unter einem Wasserstoffdruck zwischen 1 und 30 bar hydriert zur Bildung von Perindopril der Formel (I), welches man gewünschtenfalls in ein pharmazeutisch annehmbares Salz, wie das tert.-Butylaminsalz, umwandelt.

2. Syntheseverfahren nach Anspruch 1, **dadurch gekennzeichnet, dass** der Wasserstoffdruck bei der Hydrierreaktion zwischen 1 und 10 bar liegt.

3. Syntheseverfahren nach einem der Ansprüche 1 oder 2, **dadurch gekennzeichnet, dass** X₁ und X₂ jeweils ein Chloratom oder eine Imidazolyl- oder Trichlormethoxygruppe bedeuten.

4. Syntheseverfahren nach einem der Ansprüche 1 bis 3 für die Herstellung von Perindopril in Form seines tert.-Butylaminsalzes.
